# EUROPEAN PATENT SPECIFICATION

(11) **EP 2 740 513 B1**
(45) Date of publication and mention of the grant of the patent: **09.01.2019**
(21) Application number: 13186569.3
(22) Date of filing: 30.09.2013
(51) Int. Cl.: A61M 25/09

(54) **Guidewire**
Führungsdraht
Fil-guide

(30) Priority: 06.12.2012 JP 2012266896
(43) Date of publication of application: 11.06.2014
(73) Proprietor: ASAHI INTECC CO., LTD., Nagoya-shi, Aichi 463-0024 (JP)
(72) Inventor: Kosugi, Tomoki, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 361 652
- EP-A1- 2 402 051
- WO-A1-2009/039063
- JP-A- H1 176 415
- US-A1- 2004 073 141
- US-A1- 2004 167 436
- US-A1- 2010 228 151

## Description

### BACKGROUND OF THE INVENTION

The present invention relates to a guidewire that is to be inserted into a blood vessel.

### 2. Description of the Related Art

A guidewire is known as one of medical devices used for intravascular treatment such as percutaneous transluminal coronary angioplasty. A guidewire is a medical device that guides a catheter, a stent, or other devices to a target site inside a blood vessel.

Intervention from the inguen to a femoral artery (or transfemoral intervention (TFI)) used to be a popular method for inserting a guidewire into a blood vessel. However, as the size reduction of medical devices has progressed recent years, TFI has been replaced by transbrachial intervention (TBI), intervention from a brachial region to a brachial artery, or transradial intervention (TRI), intervention from a wrist to a radial artery, because the burden on patients can be reduced by using TBI or TRI.

Peripheral blood vessels located in a brachial artery, a radial artery, or other regions are thin and include a large number of bifurcations at which a main branch and a side branch bifurcate. Thus, a guidewire is difficult to smoothly arrive at a target site through peripheral blood vessels because the distal end of the guidewire may become stuck on a side branch of a peripheral blood vessel. In view of this situation, a highly steerable guidewire that includes a distal end portion having multiple curved portions is known (see, JP 11-76415 A or WO 2007-105531 A1). The guidewire according to WO 2007-105531 A1 includes three curved portions in a distal region and has a configuration that the stiffness of the curved portions increases from the distal curved portion to the proximal curved portion (in other words, the distal curved portion is the most flexible).

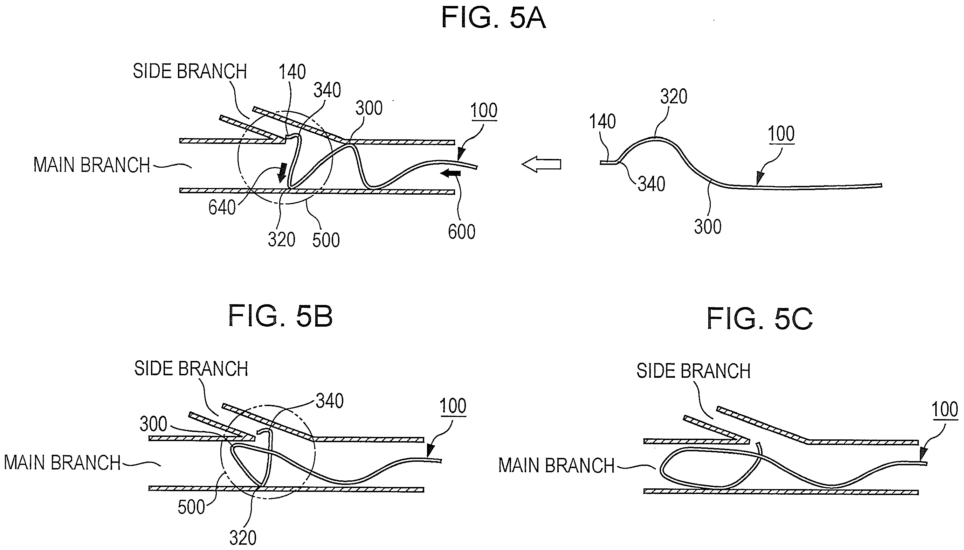

However, even when a guidewire 100 includes a distal region including a first curved portion 300, a second curved portion 320, and a third curved portion 340, a distal tip 140 of the guidewire 100 may become stuck on a side branch at a bifurcation 500 of a peripheral blood vessel (see Fig. 5A) if the angle between a main branch and the side branch is an acute angle. If the guidewire 100 is further pushed toward the distal end side while the distal tip 140 of the guidewire 100 is stuck on a side branch, the following problem would occur. The proximal-side first curved portion 300 of the guidewire 100 has a larger stiffness than the second curved portion 320, which is on the distal end side of the first curved portion 300. Thus, around the second curved portion 320 that is in contact with the side wall of the main branch, a force 600 that pushes the guidewire 100 toward the distal end side is converted into a force 640 that pushes the second curved portion 320 outward against a side wall of the main branch. Then, the guidewire 100 is unintentionally rotated around the second curved portion 320 and the distal region of the guidewire 100 is formed into a loop (see Fig. 5B). Consequently, the guidewire 100 is deformed in some cases (see Fig. 5C).

### SUMMARY OF THE INVENTION

The invention was made in view of the above circumstances and it is an object of the present invention to provide a highly steerable guidewire that is less likely to be formed into a loop even when the guidewire is pushed toward a distal end side through a peripheral blood vessel having a large number of bifurcations while a distal tip of the guidewire is stuck on a side branch of the peripheral blood vessel.

The above object is accomplished by a guidewire in accordance with claim 1. Dependent claims 2 and 3 relate to embodiments of the guidewire of claim 1.

A guidewire in accordance with the present invention includes a core shaft; and a coil body that surrounds an outer periphery of the core shaft. A distal region of the guidewire includes a first curved portion that is curved in a first direction, a second curved portion located on a distal end side of the first curved portion, the second curved portion being curved in a second direction, which is different from the first direction, and a third curved portion located on a distal end side of the second curved portion, the third curved portion being curved in a third direction, which is different from the second direction. The second curved portion has higher flexibility than the first curved portion and the third curved portion.

In the distal region of the guidewire according to the first aspect of the present invention, the second curved portion has higher flexibility than the first curved portion and the third curved portion. Thus, a portion around the second curved portion, which is flexible, can bend to a large degree even when the guidewire is pushed toward the distal end side while the distal tip of the guidewire is in a state of being stuck on a side branch of a peripheral blood vessel. With this configuration, the amount of force of pushing the guidewire toward the distal end side that is to be converted into a force of pushing the second curved portion outward against a side wall of a main branch can be reduced and the distal region can be made less likely to be rotated around the second curved portion. Consequently, the distal end of the guidewire can normally move through a main branch without being formed into a loop.

### BRIEF DESCRIPTION OF THE DRAWINGS

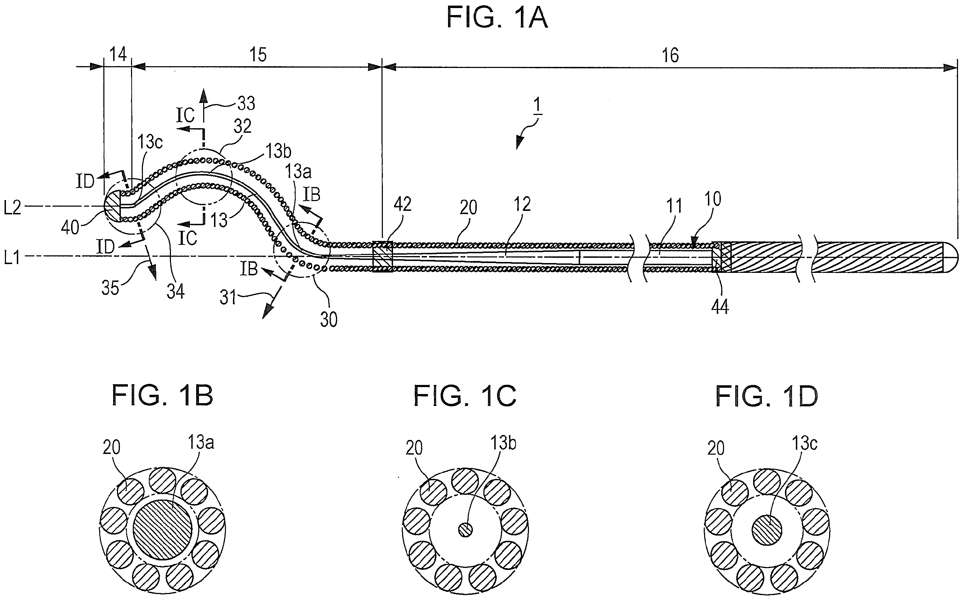

Fig. 1A illustrates the entirety of a guidewire according to an embodiment, Fig. 1B is a cross-sectional view of the guidewire taken along the line IB-IB of Fig. 1A, Fig. 1C is a cross-sectional view of the guidewire taken along the line IC-IC of Fig. 1A, and Fig. 1D is a cross-sectional view of the guidewire taken along the line ID-ID of Fig. 1A;

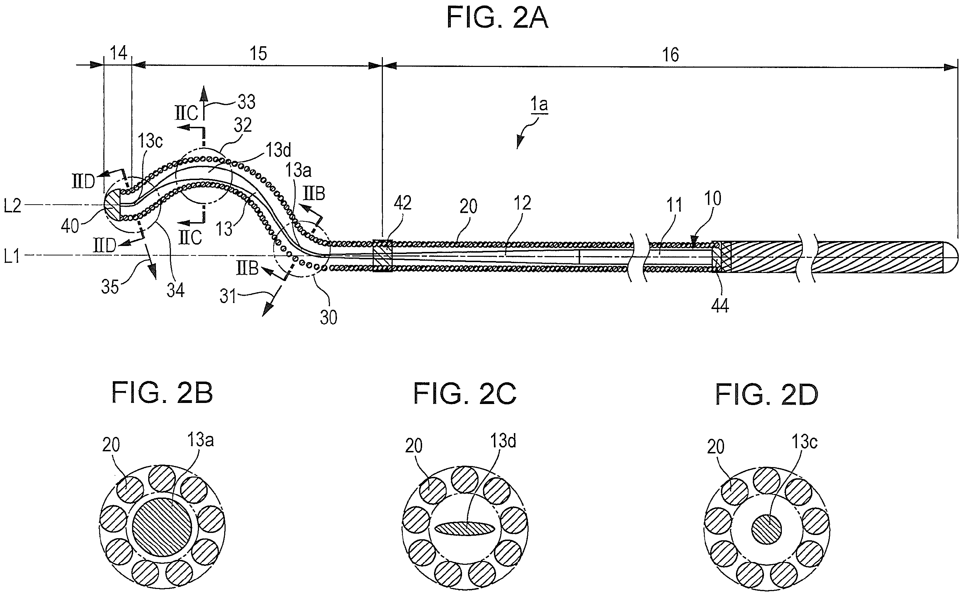

Fig. 2A illustrates the entirety of a guidewire according to another embodiment, Fig. 2B is a cross-sectional view of the guidewire taken along the line IIB-IIB of Fig. 2A, Fig. 2C is a cross-sectional view of the guidewire taken along the line IIC-IIC of Fig. 2A, and Fig. 2D is a cross-sectional view of the guidewire taken along the line IID-IID of Fig. 2A;

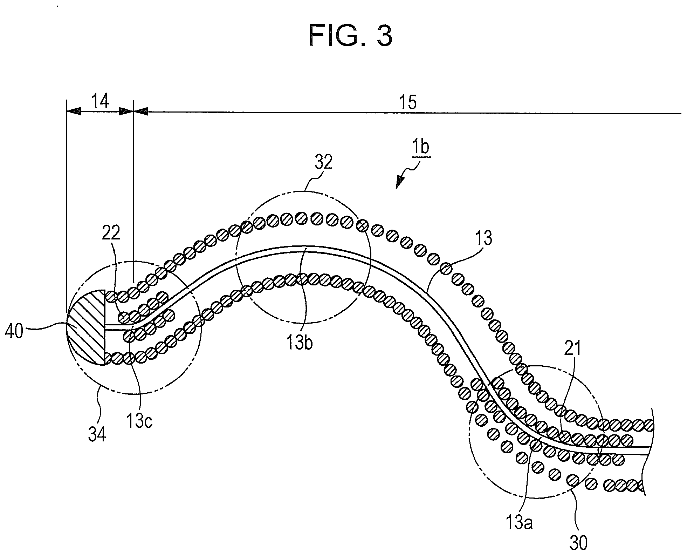

Fig. 3 is an enlarged view of a distal region of a guidewire according to another embodiment;

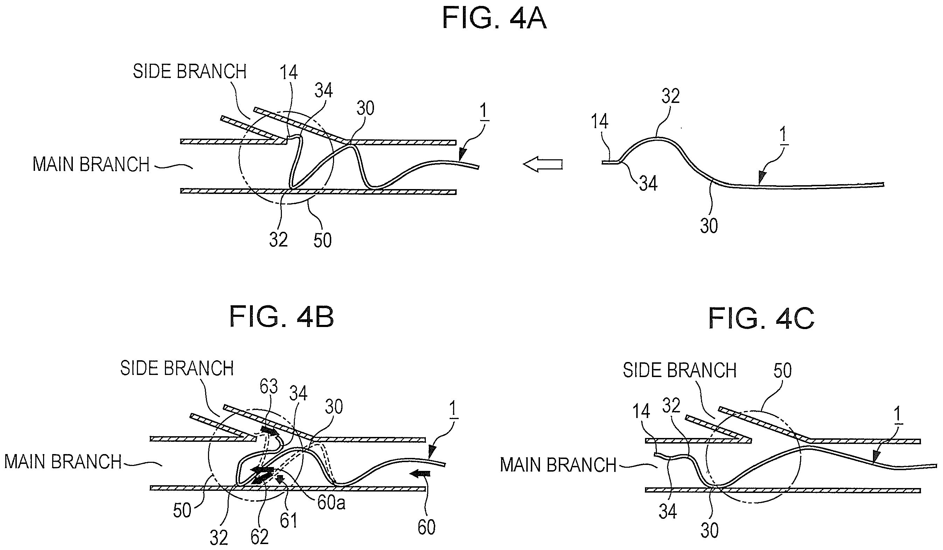

Figs. 4A to 4C illustrate a movement of the guidewire according to the embodiment inside a peripheral blood vessel, where Fig. 4A illustrates the state where the distal tip of the guidewire is stuck on a side branch, Fig. 4B illustrates the state where the guidewire is further pushed toward the distal end side while the distal tip is in the state of being stuck, and Fig. 4C illustrates the state where the distal tip of the guidewire is released from the side branch and returns to a main branch; and
Figs. 5A to 5C illustrate a movement of an existing guidewire inside a peripheral blood vessel, where Fig. 5A illustrates the state where the distal tip of the guidewire is stuck on a side branch, Fig. 5B illustrates the state where a distal region of the guidewire is formed into a loop after the guidewire is further pushed toward the distal end side while the distal tip is in the state of being stuck, and Fig. 5C illustrates the state where the guidewire is deformed.

### DESCRIPTION OF THE PREFERRED EMBODIMENTS

Referring now to Fig. 1A to Fig. 5C, a guidewire 1 according to an embodiment is described as an example. Throughout Fig. 1A to Fig. 5C, the left side is a distal end side (far side) that is inserted into a body while the right side is a proximal end side (a near side or a base side) that is manipulated by a technician such as a doctor. For ease of understanding, components such as a core shaft 10, a coil body 20, and curved portions 30, 32, and 34, which are smaller than other components and will be described below, are slightly exaggerated throughout the drawings relative to the dimensions of other components. In addition, for ease of understanding, the dimension of the guidewire 1 in the axial direction (lengthwise direction) is reduced to schematically illustrate the entire configuration and thus the ratios of the entire dimensions are not the same as the actual ones.

The guidewire 1 illustrated in Fig. 1A mainly includes a core shaft 10, a coil body 20 that surrounds the outer periphery of the core shaft 10, and a distal fixture 40 that fixes the distal end of the core shaft 10 and the distal end of the coil body 20 together.

The core shaft 10 includes a large diameter portion 11 on the proximal end side, a taper portion 12 on the distal end side of the large diameter portion 11, and a small diameter portion 13 on the distal end side of the taper portion 12. The outside diameter of the taper portion 12 decreases toward the distal end side.

The guidewire 1 also includes an intermediate fixture 42 in the taper portion 12 and a proximal fixture 44 in the large diameter portion 11 in order to fix the core shaft 10 and the coil body 20 together.

In a state where an external force is not applied to the guidewire 1, a distal region 15 of the guidewire 1 includes three curved portions, which are a first curved portion 30, a second curved portion 32, and a third curved portion 34 in order from the proximal end side. The first curved portion 30 is curved in a first direction 31. The second curved portion 32 formed on the distal end side of the first curved portion 30 is curved in a second direction 33, which is different from the first direction 31. The third curved portion 34 formed on the distal end side of the second curved portion 32 is curved in a third direction 35, which is different from the second direction 33.

Although the first direction 31 and the third direction 35 are different directions in this embodiment, the present invention is not limited to this configuration. The first direction 31 and the third direction 35 may be the same direction.

The guidewire 1 includes a distal tip 14, a distal region 15 on the proximal end side of the distal tip 14, and a linear body portion 16 on the proximal end side of the distal region 15. The distal tip 14 extends from the third curved portion 34 toward the distal end side. The distal region 15 includes the first curved portion 30, the second curved portion 32, and the third curved portion 34. An axial direction (longitudinal direction) L1 of the body portion 16 and an axial direction (longitudinal direction) L2 of the distal tip 14 are parallel to each other. The distal tip 14 is positioned higher than the first curved portion 30 but lower than the second curved portion 32. In other words, a third curved shaft portion 13c in the distal tip 14 is positioned at a height between a first curved shaft portion 13a in the first curved portion 30 and a second curved shaft portion 13b in the second curved portion 32. Thus, the distal tip 14 of the guidewire 1 extends in the axial direction L2 shifted from the axial direction L1 of the body portion 16.

Fig. 1B is a cross-sectional view of the first curved portion 30 taken along the line IB-IB of Fig. 1A. Fig. 1C is a cross-sectional view of the second curved portion 32 taken along the line IC-IC of Fig. 1A. Fig. 1D is a cross-sectional view of the third curved portion 34 taken along the line ID-ID of Fig. 1A. The outside diameter of a second curved shaft portion 13b in the second curved portion 32 is smaller than the outside diameter of a first curved shaft portion 13a in the first curved portion 30 and the outside diameter of a third curved shaft portion 13c in the third curved portion 34. On the other hand, the outside diameter of the coil body 20 in the second curved portion 32 is the same as the outside diameter of the coil body 20 in the first curved portion 30 and the third curved portion 34. For ease of understanding, the differences in outside diameter between the core shafts 13a, 13b, and 13c are noticeably exaggerated throughout Fig. 1B to Fig. 1D.

Consequently, the second curved portion 32 has higher flexibility than the first curved portion 30 and the third curved portion 34. In addition, since the outside diameter of the coil body 20 in the distal region 15 is constant, a force of a doctor pushing the guidewire 1 can be transmitted to the distal end of the guidewire 1 without being attenuated.

Now, an operation of the guidewire 1 according to the embodiment performed when the guidewire 1 is inserted into a peripheral blood vessel is described.

When the guidewire 1 is inserted into a peripheral blood vessel, the distal tip 14 of the guidewire 1 may become stuck on a side branch of a bifurcation 50 at which a main branch and the side branch bifurcates at an acute angle (see Fig. 4A). If a doctor pushes the guidewire 1 toward the distal end side while the distal tip 14 of the guidewire 1 is stuck on the side branch, a portion around the second curved portion 32 can bend to a large degree because the flexibility of the second curved portion 32 is higher than that of the first curved portion 30 and the third curved portion 34 (see Fig. 4B).

Specifically, due to the force 60 of a doctor pushing the guidewire 1 toward the distal end side (hereinafter this force is referred to as an initial distal-direction force 60), a force 60a of pushing the guidewire 1 toward the distal end side (hereinafter this force is referred to as a transmitted distal-direction force 60a) and a force 61 of pushing the guidewire 1 outward (hereinafter this force is referred to as an outward force 61) are applied to the second curved portion 32. Since the second curved portion 32 has flexibility, the second curved portion 32 can bend toward the distal end side while being in contact with the side wall of the main branch. Thus, the amount of the initial distal-direction force 60 that is converted into the outward force 61 is reduced compared to that in the case of a conventional guidewire. In other words, since the transmitted distal-direction force 60a is far larger than the outward force 61 in the second curved portion 32, the difference between the transmitted distal-direction force 60a and a resultant force 62, obtained by composition of the transmitted distal-direction force 60a and the outward force 61, is negligible. The second curved portion moves toward the distal end side by receiving the resultant force 62 and thus the distal tip 14 of the guidewire 1 returns toward the proximal end side by a proximal-direction force 63. Consequently, the guidewire 1 that is in a state of being stuck on the side branch is naturally released from the side branch.

Thus, in a portion around the second curved portion 32 of the guidewire 1 according to this embodiment, the outward force 61 exerted on the side wall of the main branch decreases further and the transmitted distal-direction force 60a accordingly increases compared to those in the case of a guidewire in which the second curved portion 32 has higher stiffness than the third curved portion 34. Thus, the guidewire 1 is less likely to be rotated around the second curved portion 32. Consequently, the distal tip 14 of the guidewire 1 released from the side branch can normally move through the main branch without being formed into a loop (see Fig. 4C).

In a state where an external force is not applied to the guidewire 1, the distal tip 14 of the guidewire 1 extends from the third curved portion 34 toward the distal end side in the axial direction L2, which is shifted from the axial direction L1 of the body portion 16 so as to be parallel to the axial direction L1. Thus, the initial distal-direction force 60 is easily transmittable to the distal tip 14.

In addition, the distal tip 14 of the guidewire 1 extends in the axial direction L2 that is shifted so as to be parallel to the axial direction L1 of the body portion 16 (in other words, the distal tip 14 of the guidewire 1 is positioned at a height between the first curved portion 30 and the second curved portion 32 and extends toward the distal end side in the axial direction L2 parallel to the axial direction L1 of the body portion 16). Thus, even in the case where the distal tip 14 of the guidewire 1 is not stuck on the side branch (in other words, in the case where the distal tip 14 of the guidewire 1 normally moves through the main branch), the guidewire 1 has the following advantages. In a peripheral blood vessel having a small diameter, the second curved portion 32 of the guidewire 1 frequently comes into contact with the side wall of a main branch. When a doctor pushes the guidewire 1 toward the distal end side while the second curved portion 32 is in contact with the side wall of a main branch, the distal-direction force and the outward force are exerted on a portion around the second curved portion 32 as in the case where the distal tip 14 of the guidewire 1 becomes stuck on a side branch. Here, by using reaction against the force of pushing the second curved portion 32 outward against the side wall of the main branch, the direction in which the distal tip 14 of the guidewire 1 extends can be slightly changed toward a direction perpendicular to the axial directions L1 and L2 of the guidewire 1. Particularly, it is preferable that the distal tip 14 of the guidewire 1 be positioned at a height closer to the second curved portion 32 than to the first curved portion 30 because, in this positioning, the direction in which the distal tip 14 of the guidewire 1 extends can be easily changed in response to the above-described reaction.

As described above, in the state where an external force is not applied to the guidewire 1, the distal tip 14 of the guidewire 1 extends from the third curved portion 34 toward the distal end side in the axial direction L2 parallel to the axial direction L1 of the body portion 16 and positioned higher than the first curved portion 30 but lower than the second curved portion 32. Thus, the direction in which the guidewire 1 moves can be flexibly changed even when the guidewire 1 moves through a sharply curved main branch of a peripheral blood vessel. Consequently, a highly steerable guidewire 1 can be provided.

Now, materials of which the components of the guidewire 1 according to this embodiment are made will be described, although the invention is not limited to these materials.

Superelastic alloy such as stainless steel (Japanese Industrial Standards (JIS) No. SUS304 or SUS316) or Ni-Ti alloy can be used for the core shaft 10 including the large diameter portion 11, the taper portion 12, and the small diameter portion 13.

The material of the coil body 20 is not particularly limited, but a stainless steel is used in this embodiment. A single coil body formed of one strand or a multi-strand coil body formed of multiple strands may be used as the coil body 20. Since a multi-strand coil body has higher breaking strength and higher flexibility than a single coil body, it is preferable to use a multi-strand coil body. A hollow coil body obtained by stranding multiple strands into a hollow shape may be used as a multi-strand coil body.

A hard solder material (aluminum alloy, silver, or gold) or a metal solder (such as Ag-Sn alloy or Au-Sn alloy) may be used as the materials of the distal fixture 40, the intermediate fixture 42, and the proximal fixture 44.

In the guidewire 1 according to the embodiment, the outside diameter of the second curved shaft portion 13b is smaller than the outside diameter of the first curved shaft portion 13a and the outside diameter of the third curved shaft portion 13c so that the second curved portion 32 has higher flexibility than the first curved portion 30 and the third curved portion 34. However, the present invention is not limited to this configuration. As illustrated in Fig. 2A and Fig. 2C, a guidewire 1a according to another embodiment may have a second curved shaft portion 13d of a flat-plate shape. When the second curved shaft portion 13d has a flat-plate shape, the second curved shaft portion 13d is made less likely to be plastically deformed. In other words, the second curved portion 32 can maintain its shape even after the guidewire 1a is used multiple times.

As illustrated in Fig. 3, a guidewire 1b according to another embodiment may include second coil bodies 21 and 22, which are different from the coil body 20, in the first curved portion 30 and the third curved portion 34. The second coil bodies 21 and 22 are disposed between the small diameter portion 13 of the core shaft 10 and the coil body 20. Specifically, in the guidewire 1b, the outer periphery of the first curved shaft portion 13a in the first curved portion 30 is covered by the second coil body 21 and the outer periphery of the third curved shaft portion 13c in the third curved portion 34 is covered by the second coil body 22, whereas the outer periphery of the second curved shaft portion 13b in the second curved portion 32 is not covered by a second coil body. Thus, the second curved portion 32 can have higher flexibility than the first curved portion 30 and the third curved portion 34.

As in the case of the guidewire 1 and the guidewire 1a, a small diameter portion 13 of a core shaft 10 of the guidewire 1b may be formed such that a first curved portion 30, a second curved portion 32, and a third curved portion 34 have different outside diameters. However, since the stiffness of the first curved portion 30 and the stiffness of the third curved portion 34 can be increased by providing the second coil bodies 21 and 22 thereto, it is preferable in view of manufacturability to manufacture the small diameter portion 13 of the core shaft 10 such that the first curved portion 30, the second curved portion 32, and the third curved portion 34 have the same outside diameter.

In the case where the second coil bodies 21 and 22 are made of the same material, the diameter of strands of the second coil body 22 only has to be smaller than that of the second coil body 21 so as to make the third curved portion 34 more flexible than the first curved portion 30. In the case where the second coil bodies 21 and 22 are made of different materials, the second coil body 22 only has to be made of a material that is more flexible than a material of the second coil body 21 so as to make the third curved portion 34 more flexible than the first curved portion 30.

Although not illustrated in Fig. 3, both ends of the second coil bodies 21 and 22 may be fixed to the small diameter portion 13 of the core shaft 10 by brazing or soldering using a hard solder material or a metal solder, as in the case of the fixtures 40, 42, and 44. Alternatively, the both ends of the second coil bodies 21 and 22 may be welded by laser or the like without using other materials.

As described above, in each of the guidewires 1, 1a, and 1b, the second curved portion 32 has higher flexibility than the first curved portion 30 and the third curved portion 34 and thus a portion around the second curved portion 32 can bend to a large degree. Consequently, the distal tip 14 of the guidewire 1, in a state of being stuck on a side branch, can be released from the side branch and the distal region 15 can be prevented from being rotated around the second curved portion 32.

## Claims

1. A guidewire (1) comprising:
a core shaft (10); and
a coil body (20) that surrounds an outer periphery of the core shaft (10),
wherein a distal region (15) of the guidewire (1) includes
a first curved portion (30) that is curved in a first direction (31),
a second curved portion (32) located on a distal end side of the first curved portion (30), the second curved portion (32) being curved in a second direction (33), which is different from the first direction (31), and
a third curved portion (34) located on a distal end side of the second curved portion (32), the third curved portion (34) being curved in a third direction (35), which is different from the second direction, **characterized in that** the second curved portion (32) has higher flexibility than the first curved portion (30) and the third curved portion (34).

2. The guidewire (1) according to Claim 1,
wherein the core shaft (13d)in the second curved portion (32) has a flat-plate shape, and
wherein an outside diameter of the coil body (20) in the second curved portion (32) is the same as an outside diameter of the coil body (20) in the first curved portion (30) and an outside diameter of the coil body (20) in the third curved portion (34).

3. The guidewire (1) according to Claim 1 or 2,
wherein a linear body portion (16) is located on a proximal end side of the distal region (15), and
wherein a distal tip (14) of the guidewire (1) extends toward a distal end side of the guidewire (1) in a direction parallel to a direction in which an axis of the body portion (16) extends.

## Patentansprüche

1. Führungsdraht (1) mit:
einem Kernschaft (10); und
einem Wicklungskörper (20), der den Außenumfang des Kernschafts (10) umgibt,
wobei ein distaler Bereich (15) des Führungsdrahts (1) aufweist:
einen ersten Krümmungsabschnitt (30), der in einer erste Richtung (31) gekrümmt ist,
einen auf der distalen Stirnseite des ersten Krümmungsabschnitts (30) liegenden zweiten Krümmungsabschnitt (32), der in einer von der ersten Richtung (31)verschiedenen zweiten Richtung (33) gekrümmt ist, und
einen auf einer distalen Stirnseite des zweiten Krümmungsabschnitts (32) liegenden dritten Krümmungsabschnitt (34), der in einer von der zweiten Richtung verschiedenen dritten Richtung (35) gekrümmt ist, **dadurch gekennzeichnet, dass**
der zweite Krümmungsabschnitt (32) eine höhere Flexibilität hat als der erste Krümmungsabschnitt (30) und der dritte Krümmungsabschnitt (34).

2. Führungsdraht (1) nach Anspruch 1,
wobei der Kernschaft (13d) in dem zweiten Krümmungsabschnitt (32) die Form einer flachen Platte hat, und
wobei der Außendurchmesser des Wicklungskörpers (20) in dem zweiten Krümmungsabschnitt (32) gleich dem Außendurchmesser des Wicklungskörpers (20) in dem ersten Krümmungsabschnitt (30) und dem Außendurchmesser des Wicklungskörpers (20) in dem dritten Krümmungsabschnitt (34) ist.

3. Führungsdraht (1) nach Anspruch 1 oder 2,
wobei auf einer proximalen Stirnseite des distalen Bereichs (15) ein linearer Körperabschitt (16) liegt, und
wobei sich eine distale Spitze (14) des Führungsdrahts (1) zu distalen Stirnseite des Führungsdrahts (1) hin parallel zur Erstreckungsrichtung der Achse des Körperabschnitts (16) erstreckt.

## Revendications

1. Fil de guidage (1) comprenant :
un arbre formant âme (10) ; et
un corps bobiné (20) qui entoure une périphérie extérieure de l'arbre formant âme (10),
dans lequel une région distale (15) du fil de guidage (1) inclut
une première portion incurvée (30) qui est incurvée dans une première direction (31),
une deuxième portion incurvée (32) située sur un côté d'extrémité distale de la première portion incurvée (30), la deuxième portion incurvée (32) étant incurvée dans une deuxième direction (33), qui diffère de la première direction (31), et
une troisième portion incurvée (34) située sur un côté d'extrémité distale de la deuxième portion incurvée (32), la troisième portion incurvée (34) étant incurvée dans une troisième direction (35), qui diffère de la deuxième direction, **caractérisé en ce que** la deuxième portion incurvée (32) a une souplesse plus élevée que la première portion incurvée (30) et la troisième portion incurvée (34).

2. Fil de guidage (1) selon la revendication 1,
dans lequel l'arbre formant âme (13d) dans la deuxième portion incurvée (32) a une forme de plaque plate, et
dans lequel un diamètre extérieur du corps bobiné (20) dans la deuxième portion incurvée (32) est le même qu'un diamètre extérieur du corps bobiné (20) dans la première portion incurvée (30) et qu'un diamètre extérieur du corps bobiné (20) dans la troisième portion incurvée (34).

3. Fil de guidage (1) selon la revendication 1 ou 2,
dans lequel une portion formant corps linéaire (16) est située sur un côté d'extrémité proximale de la région distale (15), et
dans lequel un bout distal (14) du fil de guidage (1) s'étend vers un côté d'extrémité distale du fil de guidage (1) dans une direction parallèle à une direction dans laquelle un axe de la portion formant corps (16) s'étend.
